# EUROPEAN PATENT APPLICATION

(11) **EP 4 147 665 A1**
(43) Date of publication of application: **15.03.2023**
(21) Application number: 21800108.9
(22) Date of filing: 27.04.2021
(51) Int. Cl.: A61B 34/35, A61B 34/00

(54) **MASTER MANIPULATOR GRIPPER FOR SURGICAL ROBOT**

(30) Priority: 06.05.2020 CN 202010371771
(71) Applicant: Suzhou Kangduo Robot Co., Ltd., Suzhou, Jiangsu 215153 (CN)
(72) Inventor: WANG, Jianguo, Suzhou, Jiangsu 215153 (CN); WANG, Xiaowei, Suzhou, Jiangsu 215153 (CN)
(74) Representative: Zaboliene, Reda
(86) International application number: PCT/CN2021/090203
(87) International publication number: WO 2021/223625

(57) **Abstract**

The present invention provides a master manipulator gripper for a surgical robot and belongs to the technical field of medical instruments, comprising a base, and a first handle and a second handle hinged to the base, and further comprising a touch-sensing module provided on the base, wherein the touch-sensing module is configured for sensing whether the first handle and the second handle are touched and transmitting information about whether the first handle and the second handle are touched to a distal instrument. Due to the provision of the touch-sensing module, the remote instrument can be powered on or off by sensing whether an operator contacts the handle, so that the power on or off of the remote instrument is synchronized with the operator contacting or leaving the handle, reducing the possibility of misoperation and avoiding unnecessary injury to a patient.

## Description

### Technical Field

The present invention relates to a medical instrument and, in particular, to a master manipulator gripper for a surgical robot.

### Background Art

Medical robots have unique advantages and great potential in the fields of assisted surgery, health prevention, disease diagnosis and treatment, rehabilitation and medical services. In the assisted remote minimally invasive surgery, there is a higher demand for the medical robots to achieve both precision of instructions and a very accurate master-slave response linkage.

During a remote operation, the remote operation medical system includes a remote medical robot and a control device of the robot which are coupled with each other. However, there is a difference in action steps between the existing remote medical robot and the control device, which may cause injury to a patient due to the misoperation of the operator.

### Summary of the Invention

In order to solve one of the above problems, the present invention provides a master manipulator gripper for a surgical robot, comprising a base, and a first handle and a second handle hinged to the base, and further comprising a touch-sensing module provided on the base, wherein the touch-sensing module is configured for sensing whether the first handle and the second handle are touched and transmitting information about whether the first handle and the second handle are touched to a distal instrument.

Optionally, the touch-sensing module is a capacitive touch sensor, the first handle and the second handle being capable of conducting static electricity, the capacitive touch sensor being configured for sensing static electricity on the first handle and the second handle and transmitting voltage information corresponding to the static electricity to the distal instrument.

Optionally, both of the first handle and the second handle are made of an electrically conductive material, or both of the first handle and the second handle are provided with an electrically conductive material on their surfaces.

Optionally, it further comprises a remote instrument control module, a sensing element and a moving mechanism, wherein the remote instrument control module and the sensing element cooperate with each other, the remote instrument control module is fixed on the base, and the sensing element is provided on the moving mechanism connected to the first handle and the second handle; when the first handle and the second handle are pressed, the first handle and the second handle drive the moving mechanism to move in a direction away from the remote instrument control module; when the first handle and the second handle are released, the first handle and the second handle drive the moving mechanism to move in a direction close to the remote instrument control module; and the remote instrument control module is configured for sensing information about the sensing element moving close or away and transmitting the information about moving close or away to the distal instrument.

Optionally, it further comprises a resilient connecting rod provided between the first handle and the second handle for automatically resetting the first handle and the second handle after being pressed.

Optionally, the resilient connecting rod is a spring plate.

Optionally, the remote instrument control module is a magnetic switch sensor, and the sensing element is a magnet.

Optionally, the moving mechanism comprises a moving shaft, a guide seat, a fixed seat, a first handle connecting rod and a second handle connecting rod; the guide seat is fixed on the base, a guide hole is provided on the guide seat, and the sensing element is fixed on an end of the moving shaft; an end of the moving shaft away from the sensing element passes through the guide hole and is connected to the fixed seat; a side of the first handle opposite to the second handle is hinged to one end of the first handle connecting rod, the other end of the first handle connecting rod is hinged to one end of the fixed seat close to the first handle; and a side of the second handle opposite to the first handle is hinged to one end of the second handle connecting rod, and the other end of the second handle connecting rod is hinged to the other end of the fixed seat close to the second handle.

Optionally, a fixed plate is provided on a side surface of the base, and both of the touch-sensing module and the remote instrument control module are provided on the fixed plate.

Optionally, the side surface of the base provided with the fixed plate is a first side surface, a top cover is provided on the top of the base, and the first side surface of the base is covered with an upper cover cooperating with the top cover and the base; a gap is left between the upper cover and the base, and the first handle and the second handle are adapted to protrude from the gap.

Compared with the prior art, the advantageous effects of the present invention are as follows. Due to the provision of the touch-sensing module in the present invention, the remote instrument can be powered on or off by sensing whether an operator contacts the handle, so that the power on or off of the remote instrument is synchronized with the operator contacting or leaving the handle, reducing the possibility of misoperation and avoiding unnecessary injury to a patient.

### Brief Description of the Drawings

Fig. 1 is a front view of a master manipulator gripper for a surgical robot according to the present invention;
Fig. 2 is an axial view of a master manipulator gripper for a surgical robot according to the present invention;
Fig. 3 is an axial view of a master manipulator gripper for a surgical robot with a top cover and an upper cover according to the present invention;
Fig. 4 is a structure view of a fixed seat in a master manipulator gripper for a surgical robot according to the present invention;
Fig. 5 is a structure view of a moving shaft in a master manipulator gripper for a surgical robot according to the present invention;
Fig. 6 is a structure view of a guide seat in a master manipulator gripper for a surgical robot according to the present invention.

### Description of Reference Numerals:

11-base; 111-fixed plate; 112-top cover; 113-upper cover; 12-first handle; 13-touch-sensing module; 14-remote instrument control module; 15-sensing element; 16-moving mechanism; 161-moving shaft; 162-guide seat; 163-fixed seat; 164-first handle connecting rod; 165-guide hole; 17-resilient connecting rod.

### Detailed Description of the Invention

The above and other technical features and advantages of the present invention will be described in more detail with reference to the accompanying drawings.

In addition, it should be understood in the description of the present invention that the forward direction of "X" in the drawings represents the left and, correspondingly, the reverse direction of "X" represents the right; the forward direction of "Y" represents the front and, correspondingly, the reverse direction of "Y" represents the rear; the forward direction of "Z" represents upward and, correspondingly, the reverse direction of "Z" represents downward. The orientation or positional relationships indicated by the terms "X", "Y", "Z", etc. are merely for convenience in describing and simplifying the present invention and do not indicate or imply that the device or element being referred to must have a particular orientation, be constructed and operated in a particular orientation and, therefore, should not be construed as limiting the invention.

If there is a description of "first", "second", etc. in an embodiment of the invention, the description of "first", "second", etc. is for descriptive purposes only and is not to be construed as indicating or implying relative importance or implicitly indicating the number of technical features indicated.

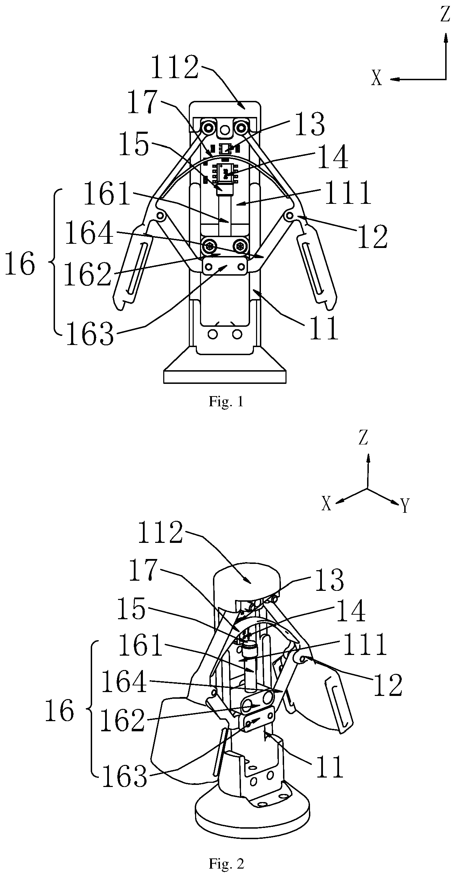

As shown in Figs. 1 and 2, a master manipulator gripper for a surgical robot according to an embodiment of the present invention includes a base 11, and a first handle 12 and a second handle hinged to two sides of the base 11. The base 11 is provided with a touch-sensing module 13 configured for sensing whether an operator's finger touches the first handle 12 and the second handle and outputting different voltages according to whether the finger touches the first handle 12 and the second handle. When the operator's finger touches the first handle and the second handle, the touch-sensing module 13 senses this information and outputs a larger voltage. The control device of the distal instrument receives the voltage information and controls the distal instrument to enable an electric motor circuit thereof to be connected, and at this time, the distal instrument can work normally according to the instruction. When the operator's finger leaves the first handle 12 and the second handle, the touch-sensing module 13 senses this information and outputs a small voltage, and the control device of the distal instrument receives the voltage information and controls the distal instrument to disconnect its electric motor circuit. At this point, the distal instrument cannot operate normally according to the instruction. Due to the provision of the touch-sensing module 13, the remote instrument can be powered on or off by sensing whether an operator contacts the handle, so that the power on or off of the remote instrument is synchronized with the operator contacting or leaving the handle, reducing the possibility of misoperation and avoiding unnecessary injury to a patient.

Specifically, the touch-sensing module 13 is a capacitive touch sensor. The first handle 12 and the second handle are made of an electrically conductive material, or the first handle 12 and the second handle are provided with an electrically conductive material on their surfaces. When an operator's finger touches the first handle 12 and the second handle, static electricity is generated on the first handle 12 and the second handle. When the operator' s finger leaves the first handle 12 and the second handle, static electricity on the first handle 12 and the second handle is reduced. The base 11 is provided with the touch-sensing module 13 configured for sensing static electricity on the first handle 12 and the second handle and outputting different voltages according to the magnitude of static electricity. When the operator's finger touches the first handle 12 and the second handle, the touch-sensing module 13 senses static electricity and outputs a larger voltage. The control device of the distal instrument receives the voltage information and controls the distal instrument to enable an electric motor circuit thereof to be connected, and at this time, the distal instrument can work normally according to the instruction. When the operator's finger leaves the first handle 12 and the second handle, the touch-sensing module 13 senses static electricity changes and outputs a smaller voltage, and the control device of the distal instrument receives the voltage information and controls the distal instrument to disconnect its electric motor circuit. At this point, the distal instrument cannot work normally according to the instruction. Since the safety switch control module 13 employs a capacitive touch sensor, the remote instrument can be powered on or off by sensing static electricity on the handle, synchronizing the powering on or off of the remote instrument with the operator contacting or leaving the handle, further reducing the possibility of misoperation.

As shown in Figs. 1 and 2, a master manipulator gripper for a surgical robot according to an embodiment of the present invention further includes a remote instrument control module 14 and a sensing element 15. The remote instrument control module 14 and the sensing element 15 cooperate with each other. The remote instrument control module 14 is fixed on the base 11. The sensing element 15 is provided on a moving mechanism 16 connected to a first handle 12 and a second handle. When the first handle 12 and the second handle are pressed, the first handle 12 and the second handle drive the moving mechanism 16 to move in a direction away from the remote instrument control module 14, so that the sensing element 15 moves in a direction away from the remote instrument control module 14. The remote instrument control module 14 senses information that the sensing element 15 is away and outputs this information. The control device of the remote instrument receives this information, and controls the remote instrument to clamp synchronously together with the first handle 12 and the second handle. When the first handle 12 and the second handle are released and the first handle 12 and the second handle are moved away from each other, the first handle 12 and the second handle drive the moving mechanism 16 to move in a direction close to the remote instrument control module 14, so that the sensing element 15 is driven to move in a direction close to the remote instrument control module 14. The remote instrument control module 14 senses the information about the sensing element 15 moving close and outputs this information. The control device of the remote instrument receives this information and controls the distal instrument to release synchronously with the first handle and the second handle. The remote instrument may be a Maryland forceps, electric hook, scissors, needle holder, etc. The provision of the remote instrument control module 14 and sensing element 15 synchronizes the releasing and clamping of the distal instrument with the action of the handle, further reducing the possibility of misoperation and avoiding unnecessary injury to the patient.

Further, a master manipulator gripper for a surgical robot according to an embodiment of the present invention further includes a resilient connecting rod 17 provided between the first handle 12 and the second handle. Specifically, the resilient connecting rod 17 is a plate-like spring plate. Due to the provision of the resilient connecting rod 17, when the operator's fingers releases them, the first handle 12 and the second handle are automatically opened, allowing the distal instrument to be released synchronously, and further reducing the possibility of misoperation and avoiding injury to the patient.

Specifically, the remote instrument control module 14 is a magnetic switch sensor, and the sensing element 15 is a magnet. When the first handle 12 and the second handle are pressed, the first handle 12 and the second handle drive the magnet to move in a direction away from the magnetic switch sensor via the moving shaft 161. The magnetic switch sensor senses the information that the magnetic field of the magnet gradually weakens, and converts the magnetic field information into a voltage output, with a smaller voltage value. The control device of the distal instrument receives the smaller voltage value, and controls the distal instrument to clamp synchronously together with the first handle 12 and the second handle. When the first handle 12 and the second handle are released and the first handle 12 and the second handle are moved away from each other, the first handle 12 and the second handle drive the magnet to move towards a direction close to the magnetic switch sensor via the moving shaft 161. The magnetic switch sensor senses the information that the magnetic field of the magnet gradually becomes stronger, and converts the magnetic field information into a voltage output, with a larger voltage value at this time. The control device of the distal instrument receives the larger voltage value, and controls the distal instrument to release synchronously with the first handle 12 and the second handle. The remote instrument control module 14 is a magnetic switch sensor, and the sensing element 15 is a magnet. The material is readily available, and it has low cost and stable performance.

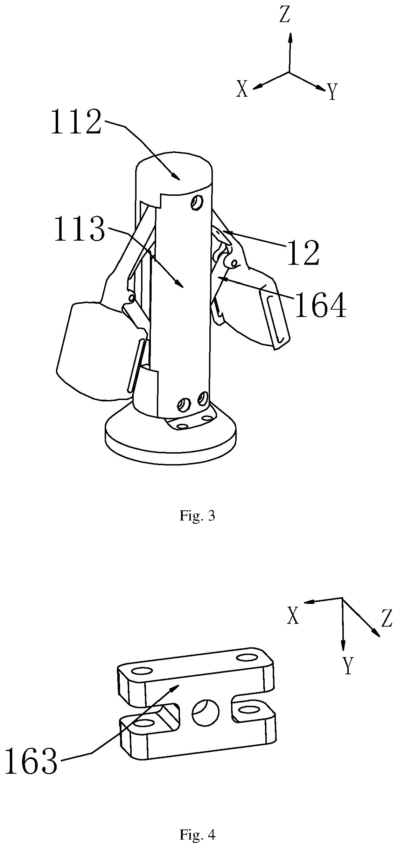

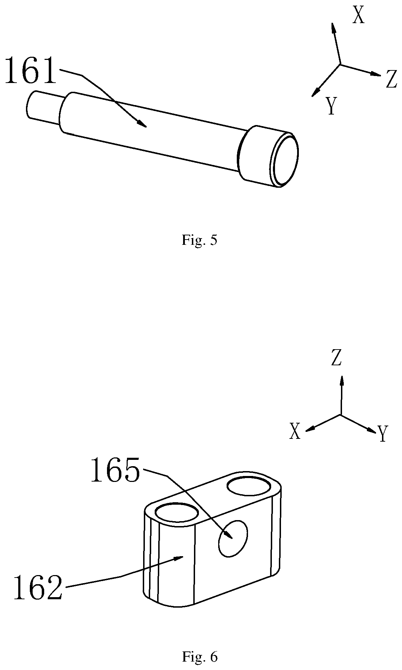

Specifically, as shown in Figs. 1 and 2, the moving mechanism 16 includes a moving shaft 161, a guide seat 162, a fixed seat 163, a first handle connecting rod 164 and a second handle connecting rod. The structure of the fixed seat 163 is as shown in Fig. 4. The structure of the moving shaft 161 is as shown in Fig. 5. The guide seat 162 is fixed on the base 11. As shown in Fig. 6, a guide hole 165 is provided in the guide seat 162. The sensing element 15 is fixed on one end of the moving shaft 161, and the other end of the moving shaft 161 away from the sensing element 15 passes through the guide hole 165 and is connected to the fixed seat 163. A side of the first handle 12 opposite to the second handle is hinged to one end of the first handle connecting rod 164, and the other end of the first handle connecting rod 164 is hinged to one end of the fixed seat 163 close to the first handle 12. A side of the second handle opposite to the first handle 12 is hinged to one end of the second handle connecting rod, and the other end of the second handle connecting rod is hinged to the other end of the fixed seat 163 close to the second handle. When the first handle 12 and the second handle are pressed, the first handle 12 and the second handle move the sensing element 15 to a direction away from the remote instrument control module 14 via the moving shaft 161. When the first handle 12 and the second handle are released, the first handle 12 and the second handle move the sensing element 15 to a direction close to the remote instrument control module 14 via the moving shaft 161.

Specifically, a fixed plate 111 is provided on a first side surface of the base 11, and a second side surface departs away from the first side surface. The touch-sensing module 13 and the remote instrument control module 14 are both provided on the fixed plate 111. The guide seat 162 and the fixed plate 111 are located on the same side surface. As shown in Fig. 3, a top cover 112 is provided on the top of the base 11. The first side surface of the base 11 is covered with an upper cover 113 cooperating with with the top cover 112 and the base 11. A gap is left between the upper cover 113 and the base 11, and and the first handle 12 and the second handle extend outside the gap.

When the master manipulator gripper for the surgical robot of the present invention is in use, and the operator touches the first handle 12 and the second handle, the touch-sensing module 13 transmits information to the remote instrument, so that the power source of the remote instrument is synchronously switched on, and the remote instrument is in a workable state. When the operator presses the handle, the remote instrument control module 14 senses this information and outputs the information. When the control device of the distal instrument receives this information, the control device of the distal instrument controls the distal instrument to clamp synchronously together with the first handle 12 and the second handle and perform a surgery. When the operator's hands release the first handle 12 and the second handle, the remote instrument control module 14 senses this information and outputs it, and the control device of the distal instrument receives this information and controls the distal instrument to release synchronously. When the operator's hand leaves the first handle 12 and the second handle, the touch-sensing module 13 transmits information to the remote instrument, so that the power source of the remote instrument is synchronously disconnected and the remote instrument is inoperative.

Although the present disclosure has been described above, the scope of protection of the present disclosure is not limited thereto. Various changes and modifications may be made by those skilled in the art without departing from the spirit and scope of this disclosure, and such changes and modifications are intended to fall within the scope of this disclosure.

## Claims

1. A master manipulator gripper for a surgical robot, comprising a base (11), and a first handle (12) and a second handle hinged to the base (11), and further comprising a touch-sensing module (13) provided on the base (11), wherein the touch-sensing module (13) is configured for sensing whether the first handle (12) and the second handle are touched and transmitting information about whether the first handle (12) and the second handle are touched to a distal instrument.

2. The master manipulator gripper for a surgical robot according to claim 1, wherein the touch-sensing module (13) is a capacitive touch sensor, the first handle (12) and the second handle being capable of conducting static electricity, the capacitive touch sensor being configured for sensing static electricity on the first handle (12) and the second handle and transmitting voltage information corresponding to the static electricity to the distal instrument.

3. The master manipulator gripper for a surgical robot according to claim 2, wherein both of the first handle (12) and the second handle are made of an electrically conductive material, or both of the first handle (12) and the second handle are provided with an electrically conductive material on their surfaces.

4. The master manipulator gripper for a surgical robot according to claim 1, further comprising a remote instrument control module (14), a sensing element (15) and a moving mechanism (16), wherein the remote instrument control module (14) and the sensing element (15) cooperate with each other, the remote instrument control module (14) is fixed on the base (11), and the sensing element (15) is provided on the moving mechanism (16) connected to the first handle (12) and the second handle; when the first handle (12) and the second handle are pressed, the first handle (12) and the second handle drive the moving mechanism (16) to move in a direction away from the remote instrument control module (14); when the first handle (12) and the second handle are released, the first handle (12) and the second handle drive the moving mechanism (16) to move in a direction close to the remote instrument control module (14); and the remote instrument control module (14) is configured for sensing information about the sensing element (15) moving close or away and transmitting the information about moving close or away to the distal instrument.

5. The master manipulator gripper for a surgical robot according to claim 4, further comprising a resilient connecting rod (17) provided between the first handle (12) and the second handle for automatically resetting the first handle (12) and the second handle after being pressed.

6. The master manipulator gripper for a surgical robot according to claim 5, wherein the resilient connecting rod (17) is a spring plate.

7. The master manipulator gripper for a surgical robot according to claim 4, wherein the remote instrument control module (14) is a magnetic switch sensor, and the sensing element (15) is a magnet.

8. The master manipulator gripper for a surgical robot according to claim 4, wherein the moving mechanism (16) comprises a moving shaft (161), a guide seat (162), a fixed seat (163), a first handle connecting rod (164) and a second handle connecting rod; the guide seat (162) is fixed on the base (11), a guide hole (165) is provided on the guide seat (162), and the sensing element (15) is fixed on an end of the moving shaft (161); an end of the moving shaft (161) away from the sensing element (15) passes through the guide hole (165) and is connected to the fixed seat (163); a side of the first handle (12) opposite to the second handle is hinged to one end of the first handle connecting rod (164), the other end of the first handle connecting rod (164) is hinged to one end of the fixed seat (163) close to the first handle (12); and a side of the second handle opposite to the first handle (12) is hinged to one end of the second handle connecting rod, and the other end of the second handle connecting rod is hinged to the other end of the fixed seat (163) close to the second handle.

9. The master manipulator gripper for a surgical robot according to claim 4, wherein a fixed plate (111) is provided on a side surface of the base (11), and both of the touch-sensing module (13) and the remote instrument control module (14) are provided on the fixed plate (111).

10. The master manipulator gripper for a surgical robot according to claim 9, wherein the side surface of the base (11) provided with the fixed plate (111) is a first side surface, a top cover (112) is provided on the top of the base (11), and the first side surface of the base (11) is covered with an upper cover (113) cooperating with the top cover (112) and the base (11); a gap is left between the upper cover (113) and the base (11), and the first handle (12) and the second handle are adapted to protrude from the gap.
